(19) Europäisches Patentamt
European Patent Office
Office européen des brevets

(11) **EP 4 155 341 A1**

(12) **EUROPEAN PATENT APPLICATION**

(43) Date of publication:
**29.03.2023 Bulletin 2023/13**

(21) Application number: **21306333.2**

(22) Date of filing: **27.09.2021**

(51) International Patent Classification (IPC):
**C08J 11/10** (2006.01)    **B29C 48/00** (2019.01)
**B29B 17/02** (2006.01)    **C07C 4/22** (2006.01)

(52) Cooperative Patent Classification (CPC):
(C-Sets available)
**B29C 48/00; C07C 67/00; C08J 11/10;**
C08J 2300/30; C08J 2325/06; C08J 2333/12

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Japan Steel Works Europe GmbH**
  **40217 Düsseldorf (DE)**
• **ARKEMA FRANCE**
  **92700 Colombes (FR)**

(72) Inventors:
• **TOJO, Makoto**
  **40217 DÜSSELDORF (DE)**
• **KAKIZAKI, Jun**
  **40217 DÜSSELDORF (DE)**
• **DUBOIS, Jean-Luc**
  **69390 MILLERY (FR)**

(74) Representative: **Lavoix**
  **2, place d'Estienne d'Orves**
  **75441 Paris Cedex 09 (FR)**

(54) **GRADE-DEPENDENT DEPOLYMERIZATION PROCESS AND ITS USE FOR RECYCLING PLASTICS**

(57) The present application concerns a depolymerization process that can discriminate the grade of the polymer that is being processed and that can adjust its working conditions to optimize its yield depending on said grade of polymer. It also concerns the recycling of plastics comprising said depolymerization.

(52) Cooperative Patent Classification (CPC): (Cont.)

C-Sets
**C07C 67/00, C07C 69/54**

**Description**

The project leading to this application has received funding from the European Union's Horizon 2020 research and innovation programme under grant agreement No 820687.

The present invention concerns the recycling of waste plastics, in particular by depolymerization, in particular by pyrolysis.

In this process, sorted plastics waste is broken down into monomers (basic building blocks of the polymers), so that they can be fed back into the plastic production.

Plastics comprise mixtures of different polymers that may be depolymerized according to different conditions. Additionally, polymers may have different grades which in turn may impact the depolymerization conditions as all grades do not necessarily depolymerize in a similar manner.

For example in the case of poly(methyl methacrylate) (PMMA), not all the PMMA grades give the same recycled monomer (recycled methyl methacrylate, rMMA) purity. Some PMMAs like the cast-grade PMMA give in general a very high purity rMMA. There are usually no co-monomers, and the product is producing little amount of solid residues and methyl isobutyrate. Other grades like the extrusion and injection PMMA grades generate methyl and/or ethyl acrylates used as co-monomers. Ethyl acrylate is especially troublesome as it cannot be separated by distillation from MMA. The presence of these impurities reduces the performance of the unit and leads to losses at the distillation/purification downstream.

Despite the variety of plastics and their constituting polymers, their recycling is however to be conducted in a single unit.

In any case, it is desirable to maximize the productivity of the depolymerization unit as the costs of the depolymerization unit (capital cost) is important in the overall cost structure of plastics waste recycling units.

It is also desirable to identify the type of materials being processed at any time and to adapt the depolymerization conditions continuously based on the data that would be collected when feeding the unit.

There is also a need to provide a process that can direct the fed materials and/or the products in order to segregate the streams, and avoid to contaminate the best quality rMMA with low quality rMMA.

It is further desirable to adapt the depolymerization conditions to the cost of energy. For example, the price of electricity may be variable (e.g. at certain times of the year or of day or depending on the availability of renewable electricity). The depolymerization process may use electric energy for the engine and electric heater. When the electricity is cheap, there would be an advantage to process more material. Therefore, another problem to solve is also to adapt the process conditions based on the electricity price.

It is thus also desirable to adapt the process conditions to the availability of renewable electricity.

At last but not least, it is paramount to reduce the fossil energy consumption, and to minimize the environmental footprint ($CO_2$ emissions for example) of the recycling.

It is therefore desirable to provide a versatile depolymerization process that is able to discriminate the materials being processed and is self-adjusting to the fed material in order to achieve the best yields.

These are some aims achieved by the present invention.

In particular, it has been discovered that different grades of polymers consume different amounts of mechanical and heating energies.

More particularly, depending on the grade of the polymers that are processed, it was surprisingly found that at constant (maximal) heating energy, the depolymerization productivity (typically the weight of produced MMA over time) can be improved by increasing the feeding rate when the consumed mechanical energy increases.

According to a first object, the present invention concerns a process for depolymerizing a polymer or polymers mixture, wherein said polymer(s) may have various grades, said process comprising the steps of :

feeding the polymer(s) to one or more processing unit(s) ;

mechanically processing and heating the polymer(s) in the processing unit(s) configured for mechanically processing and heating said polymer(s) such as to melt and depolymerize the polymer(s);

measuring the heating power and/or energy consumed by said one or more processing unit(s) and measuring the mechanical power and/or energy consumed by said one or more processing unit(s) ; and

adjusting the feeding rate of the polymer(s) into the processing unit(s) depending on the measured consumed heating energy and/or the consumed mechanical energy.

In particular, the present invention allows to optimize the depolymerization conditions (such as the feeding rate, screw rotation etc..) depending on the composition of the fed plastics that are being processed.

According to an embodiment, the depolymerisation process is limited by the heating energy at a given temperature. The heating energy that can be provided increases with the temperature, provided that the unit typically adjusts to stabilize when temperature changes (while it has an immediate response with the mechanical energy). Accordingly, the depolymerization is operated at constant heating energy, at the maximal capacity of the heating energy that can be supplied by the system.

When the material that is fed changes, the unit can detect it as there is a variation of the mechanical energy consumed. When the total energy (Heating + Mechanical Energy) remains constant, an increase of the mechanical energy means that the heating energy decreases. As the system operates at the maximal capacity of the heating energy, this allows an increase of the fed material (ie) an increase of the feeding rate.

According to an embodiment, the feeding rate is increased when the measured consumed mechanical energy increases.

On the other hand, when the unit detects that the mechanical energy consumption decreases, it means the material that is fed changes, with e.g. a decrease in cast PMMA, such as a shift from cast to extrusion PMMA. In this case, the demand for heating energy will increase, and it is appropriate to decrease the feeding rate.

Therefore, conversely, when the mechanical energy consumed decreases, then the feeding rate is decreased, to keep the heating energy consumed constant and ensure high depolymerization rate.

In particular, this may apply when said polymer has at least one grade requiring more mechanical energy compared with other grades of said polymer. Under such circumstances, the feeding rate may be adjusted as a function of the content of the grade in the polymer or polymer mixture.

More specifically, the feeding rate may be increased when the content in said grade of said polymer in the polymer or polymers mixture requiring more mechanical energy compared with other grades of said polymer increases. Feeding rates may typically be comprised between 30 and 2000 kg/hour, preferably between 30 and 1000 kg/hour, more preferably between 100 and 500 kg/hour, for example between 30 and 500 kg/hour.

As used herein, a polymer refers to a material consisting of macromolecules composed of repeating units that may be the same (monomers) or different (co-monomers).

The term "polymers mixture" refer to a material consisting of various polymers or various grades that may differ from e.g. their size or molecular mass, their constituting units, or the arrangement thereof.

The term "polymer" means either a copolymer or a homopolymer. A "copolymer" is a polymer grouping together several different monomer units and a "homopolymer" is a polymer grouping together identical monomer units.

The term "monomer" means a molecule which can undergo a polymerization.

The term "polymerization" as used herein relates to the process for converting a monomer or of a mixture of co-monomers into a polymer.

The term « depolymerization » refers to the process for converting a polymer or polymers mixture into their constituting monomers or co-monomers.

A polymer may exist in several grades in particular when it may be synthesized by a variety of routes. Various grades of a polymer may have the same or different molecular weight and/or composition in terms of monomers.

According to an embodiment, said polymer(s) to be depolymerized is(are) chosen from PMMA (Poly(methyl methacrylate)) , polystyrene (PS)., PolyLactic acid (PLA, PolyHydroxyAlkanoate (PHA), ...., more particularly said polymer(s) may be chosen from PMMA and polystyrene (PS), still more particularly PMMA.

PMMA refers to a thermoplastic homo- or copolymer of methyl methacrylate (MMA) that comprises at least 50%, preferably at least 60%, advantageously at least 70% and more advantageously at least 80% by weight of methyl methacrylate.

PMMA exists in various grades :

Cast PMMA is a form of PMMA that is formed by casting methyl methacrylate, mixed with initiators and possibly other additives into a form or mold. Cast PMMA has a high molecular weight and high purity, but cannot be recycled mechanically as it depolymerizes at a temperature close to its processing temperature.

Injection (also referred to as "Inj") and extrusion (also referred to as "XT") PMMAs are produced with co-monomers which can be ethylacrylate or methylacrylate for example. Those co-monomers inhibit the depolymerization at low temperature so that the products can be injected or extruded. In theory, these products could be mechanically recycled. The polymers have also a lower molecular weight than Cast PMMA.

There are also PMMA grades that are cross-linked, or contain additives to improve the shock resistance.

In the case of PMMA, it was surprisingly found that cast PMMA consumes more mechanical energy than other grades of PMMA. Consequently, when the fed material comprises cast PMMA, more material can be processed at constant heating energy. As a result, the feeding rate of the polymer(s) to be processed can be increased. In other terms, the more cast PMMA, the higher the feeding rate.

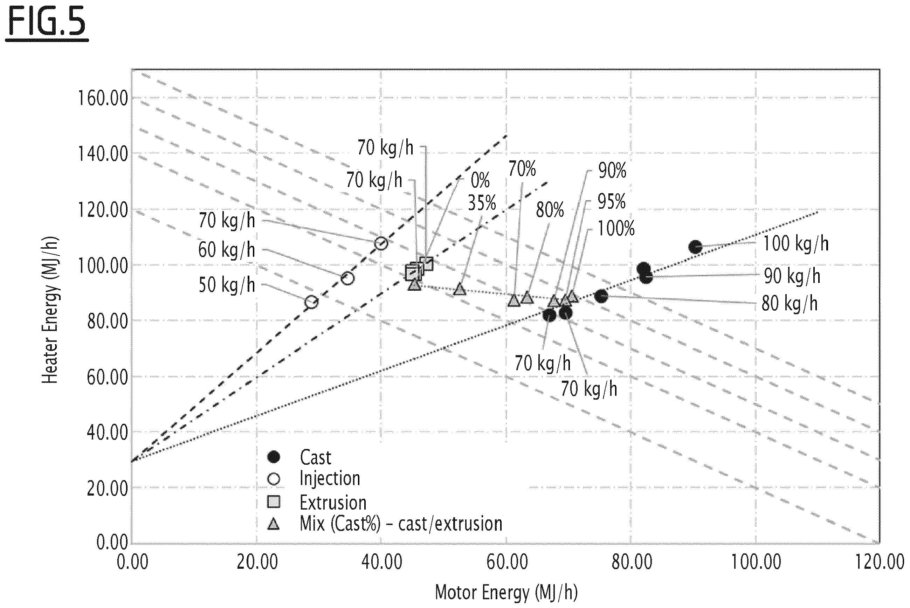

Thus, according to an embodiment, the feeding rate may be increased when the content in cast PMMA in said PMMA (such as mixed PMMA including both cast and extrusion PMMA) or polymers mixture increases. In particular, it was found according to the invention that the mechanical energy consumption changes (ie decreases) in a continuous fashion when the content of cast PMMA decreases in the fed mixed cast/extrusion PMMA. This is illustrated in particular in Figure 5 (triangles).

According to a preferred embodiment, the polymer(s) to be depolymerized include cast PMMA.

The polymer(s) or mixtures to be processed may be waste plastics of various compositions that may include additives in addition to the polymer(s).

Typically, the polymer(s) to be depolymerized are in the form of granules, pellets, crushed particles, powder or liquids like PMMA syrup (which is containing MMA oligomers and polymers dissolved in MMA), when fed into the system.

As used herein, the feeding step refers to the introduction of the polymer(s) to be depolymerized into the processing unit. According to an embodiment, this feeding step merely moves the fed material into the processing step. This can be done by various means configured to this end, such as feeders defined below.

Typically, this step does not substantially heat or process the polymer(s) that are fed.

"Processing", as used herein, refers to the heating and mechanically processing, such as extruding the polymer(s), so as to melt and depolymerize them into their respective (co)monomers.

The "processing unit(s)" as used herein refers to the means configured to process the polymer(s) as defined above.

The processing and heating step is typically conducted at temperatures higher than the melting temperature of the polymers that are being processed. Generally, the temperature may be higher than 250°C, preferably higher than 400°C. When the processing unit is an extruder, the temperature is typically comprised between 450°C and 550°C; when the processing unit(s) includes a fluidized bed reactor, the temperature of the bed could be as high as 800°C.

The processing unit(s) typically include a unit configured for melting and depolymerizing the polymer(s).

Such means include twin screws, such as twin screws extruders and electric heaters positioned around the extruder, or heated twin screws where the heater is located inside the screws as in the case of Auger screws, so as to heat, melt and depolymerize the extruded polymer(s). Typical screws diameters depend on the system scaling, and on the type of the screws that is considered (for example, Auger screws have bigger diameters than intermeshing screws). Typical twin screws diameters may be comprised between 40 and 500 mm, such as 40 and 100 mm.

Alternatively, the processing unit can comprise a pre-reactor and reactor assembly, wherein the pre-reactor may provide mechanical and heating energy and may include an extruder and melter, whereas the reactor is the tank where the actual depolymerisation occurs under heating, such as a pyrolysis tank such as fluid bed reactor, stirred tank and auger screw.

As used herein, the "mechanical energy" refers to the total mechanical energy consumed for the depolymerisation. It is essentially provided by the processing unit (in particular by the twin screws extruder, and pre-reactor as defined above) although small amounts of mechanical energy may be provided by the feeding unit.

The value of the consumed mechanical energy can be obtained in various ways.

It can be obtained from the electricity consumption of the electric engine. This electricity consumption is reported as kWh, and is divided by the time interval between 2 acquisitions to have an instant energy consumption. The data can be averaged by calculating a mobile average value using previously recorded data on more than 25 data sets, preferably more than 100, and more preferably more than 200 data. For example, the value can be calculated as linearization of a 200 data set over time.

The mechanical energy [kwh] is indicated at invertor panel of motor, and recorded continuously.

Alternatively, the mechanical energy [kwh] can also be measured by torque measurement sensor which is installed at the coupling between motor and gear box, or at screw connection parts.

Also, the mechanical energy [kwh] can also be measured by electric power meter which is installed at motor.

As provided by the invention, it was found that some polymer grades such as cast PMMA may consume more mechanical energy than others.

As used herein, the "heating energy" refers to the total heating energy consumed for the depolymerisation. It is essentially provided by the processing unit (in particular by the heater, or by the pre-reactor and reactor). The value of the consumed heating energy is obtained from the electricity consumption on each individual barrel along the screw. The data are then calculated similarly as for the mechanical energy. Typically, the heating energy [kwh] is calculated by using the heater output [%], on the basis of the following relation:

$$\text{heater energy [kwh]} = \text{Rated power [kW]} \times \text{heater output [\%]}$$

Alternatively, the heating energy [kwh] can also be measured by electric power meter which is installed at control panel.

According to the invention, the heating energy that is consumed by the process may be kept as a constant corresponding to the maximal heating capacity of the system, at a given temperature. When the operating temperature increases, the heating energy is also increased.

According to another object, the present invention also concerns a process for recycling plastics or plastics mixtures, where said plastics comprise polymers and where said process comprises the depolymerizing process of the invention as defined above.

Typically, said plastics comprise polymers and additives, such as fillers, pigments, plasticizers....

According to an embodiment, the process of the invention leads to various advantages to improve the overall yield of the depolymerization and/or to decrease the costs.

When the process identifies an increase (or a decrease) of the consumed mechanical energy, this leads to an increase (or a decrease) of the feeding rate to keep the heating energy constant, and in turn, an increase (or a decrease) the consumed electricity.

As a result, the process can advantageously be implemented at times when the electricity is cheaper. Similarly, when the process identifies an increase or decrease of the consumed mechanical energy, this means that the content of the fed polymer(s) mixture varies, in particular in terms of polymers grades. The process can thus be optimized to direct the fed materials and/or the resulting products in order to segregate the streams of recycled monomers to improve the quality of the resulting recycled monomers.

APPARATUS

According to a still further object, the present invention also concerns a depolymerization system comprising

- One or more feeder(s);
- One or more processing unit(s) configured for heating and mechanically processing the polymer(s) such as to melt and depolymerize the polymer(s) ;
- a sensor for measuring the heating energy consumed by the processing unit(s) ;
- a sensor for measuring the mechanical energy consumed by the processing unit(s) ;
- a control loop configured to control the one or more feeder(s) to adjust a feeding rate as a function of the measured consumed mechanical energy and/or the measured consumed heating energy.

Optionally, the system can also comprise a sensor for the pressure

Feeder(s) include in particular screw feeders, conveyors, belts, vibratory feeders, etc...Screw feeders are preferred herein. Usually, feeders as defined herein are not heated, nor made to heat the polymer. Preferably gravimetric feeders are used.

According to an embodiment, the processing unit(s) may include means for heating and mechanically processing the polymer(s). Typically, processing unit(s) include extruders, such as heated extruders, more particularly a heated screw extruder configured for melting and heating the polymer(s) with heating energy and/or mechanical energy. They may include twin screw extruders, where the screw is internally heated or where the heater is located around the walls of the extruder.

Processing units may also include assemblies of pre-reactors and reactors, where pre-reactors include for example melters and screw-feeders, and reactors include pyrolysis tanks such as fluid beds, stirred tanks, and auger screws.

The detection of an increase of the consumed mechanical energy is typically associated with the increased processing of certain polymers grades, such as cast grades, more particularly cast PMMA. Therefore, when the unit detects an increase in mechanical energy consumed, this is usually associated with a shift towards higher content in cast PMMA. In this case, the heating energy decreases and the unit responds in increasing the feed rate, in order to keep the heating energy constant. This is also beneficial to ensure enough polymer is fed into the extruder.

Conversely, when the unit detects a decrease in mechanical energy consumed, this is usually associated with a reverse shift, such as from cast to extrusion or injection grade PMMA. In that case, the unit responds in decreasing the feed rate, in order to keep the heating energy constant, and ensure high depolymerization rate.

The sensors may measure the energy consumed by processing unit(s) such as the screws rotation and the heaters, as instant values, average including standard deviation over a limited period.

The sensors may include control panels to continuously read the heating and mechanical energy consumption.

The control loop is configured to increase the screw rotation speed and/or the feeding rate of the fed polymers when at least one sensor measures *inter allia* an increase of the mechanical energy consumed by the processing unit(s) (and a decrease, when it detects a decrease in mechanical energy) in order to keep the heating energy constant, at a given value for a given temperature.

According to the invention, the feeding rate may be increased at the feeder(s) by the control loop when such an increase of the consumed mechanical energy is measured by the sensor. The feeding rate may typically be increased by increasing parameters such as screw speed, flowrate, etc...

BRIEF DESCRIPTION OF THE DRAWINGS

Figures

Figure 1 shows the heater energy as a function of the motor energy for various PMMA grades and illustrates the discrimination between Cast and Extruded PMMA grades.

Figure 2 illustrates the gross energy and net Energy (Gross minus heat loss) calculated for all the tests (1 to 27). All products included.

Figure 3 is a schematic representation of the depolymerization system of the invention.

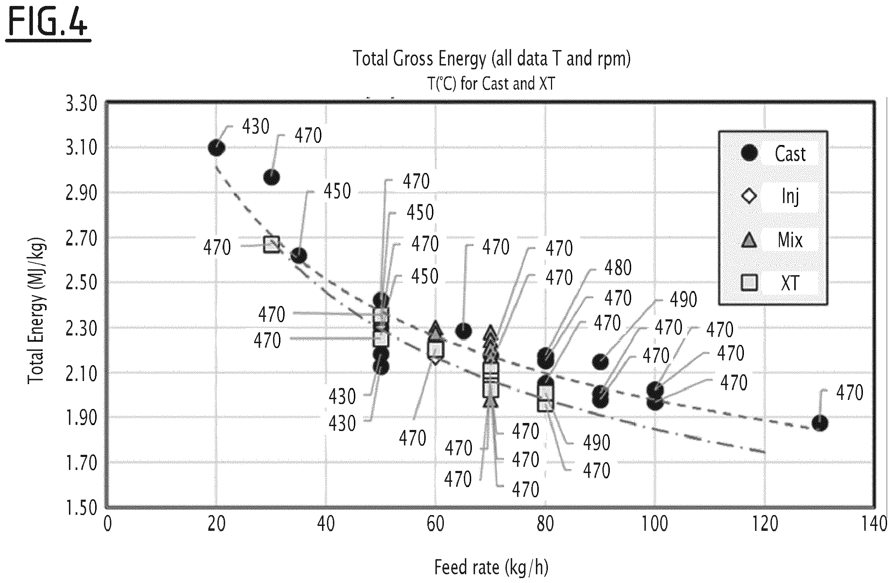

Figure 4 illustrates the total energy consumed (mechanical+heating energies) reported versus the feeding rate for all the conditions for cast-, Injection-, mixed and extrusion (XT)-PMMA material.

Figure 5 illustrates the heating energy as a function of the motor energy. Black circles: cast materials at 470 °C, 800 rpm. Hatched squares: extrusion grades in other conditions. Gray triangles: mixed Cast and extrusion materials at 470 °C, 800 rpm and 70 kg/h, with cast content indicated. Open circles: Injection grade at 470 °C and 800 rpm (rotations per minute).

Doted line from top left to bottom right are indicating constant total energy (mechanical + heating). Lines from bottom left to top right are linearized data for data points of similar conditions (cast at 470 °C, 800 rpm and varying feeding rates) - black circles; (extrusion, 470 °C, 800 rpm, varying feeding rates) - gray square; (injection, 470 °C, 800 rpm, varying feeding rates) - white circles.
The figure illustrates that based on energy consumption data, the control loop can detect the type of material being processed.

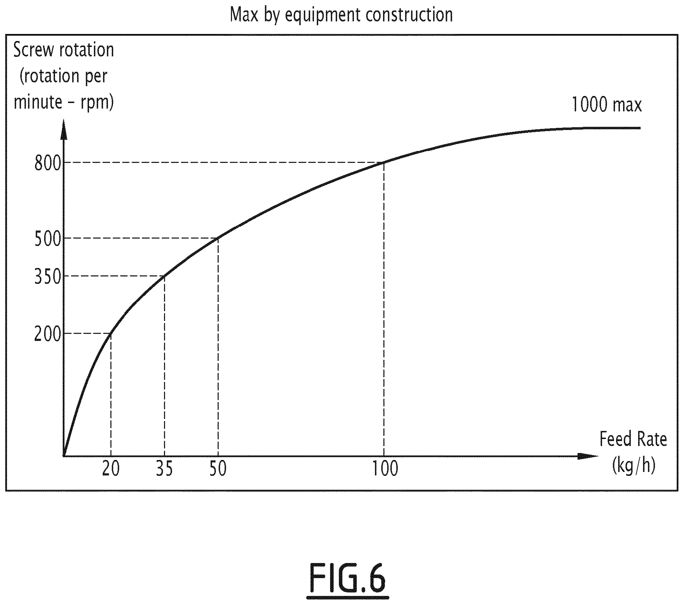

Figure 6 shows a representative variation of the maximum screw rotation as a function of the feed rate (in kg/h).

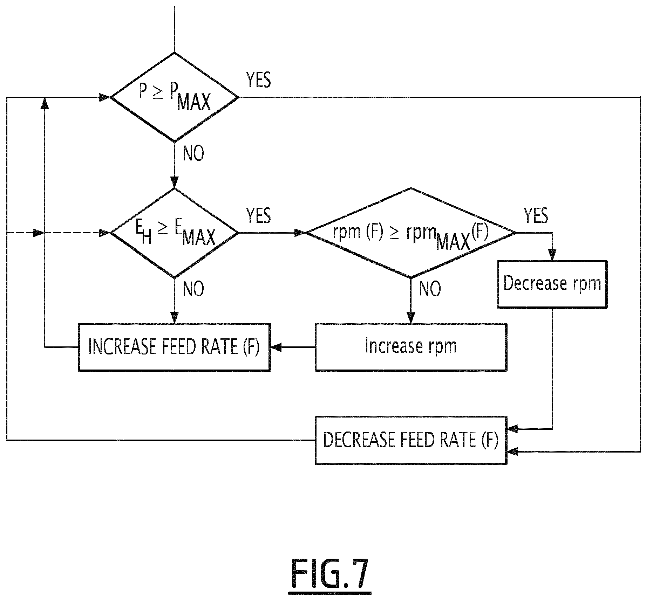

Figure 7 is a schematic diagram of control loop 1 where $E_H$ refers to the heating energy, rpm refers to the screw rotation (in rpm), P is the pressure (measured at the end of the screw) and F is the feeding rate. The dotted line is optional as the pressure sensor is optional (in this case, the pressure measurement would be avoided and the loop would directely go to EH>=E max).

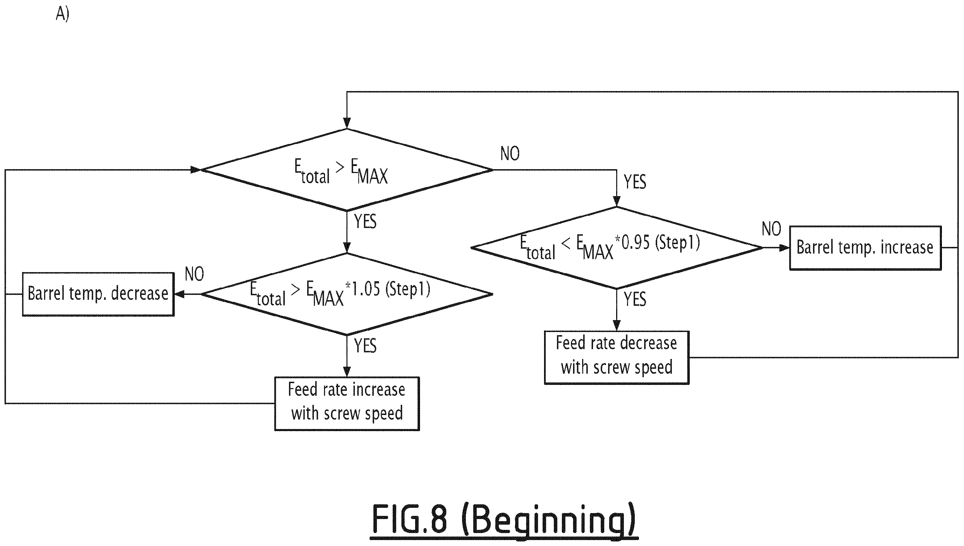

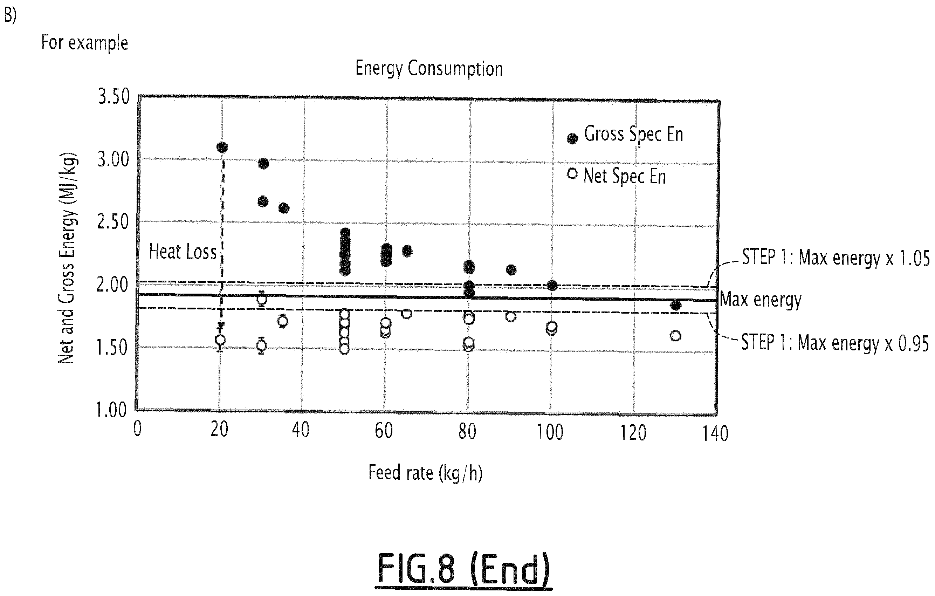

Figure 8 illustrates an alternative control loop. Figure 8A is a schematic diagram of control loop 2 where $E_H$ refers to the heating energy, $E_M$ refers to the mechanical energy, $E=E_H+E_M$. $E_{Max}$ is the limit energy for depolymerization; rpm refers to the screw rotation (in rpm), P is the pressure (measured at the end of the screw) and F is the feeding rate.

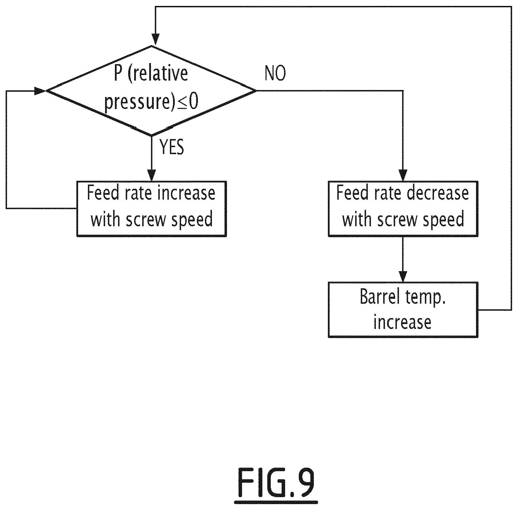

Figure 9 illustrates an alternative control loop 3.

DETAILED DESCRIPTION OF SPECIFIC EMBODIMENTS

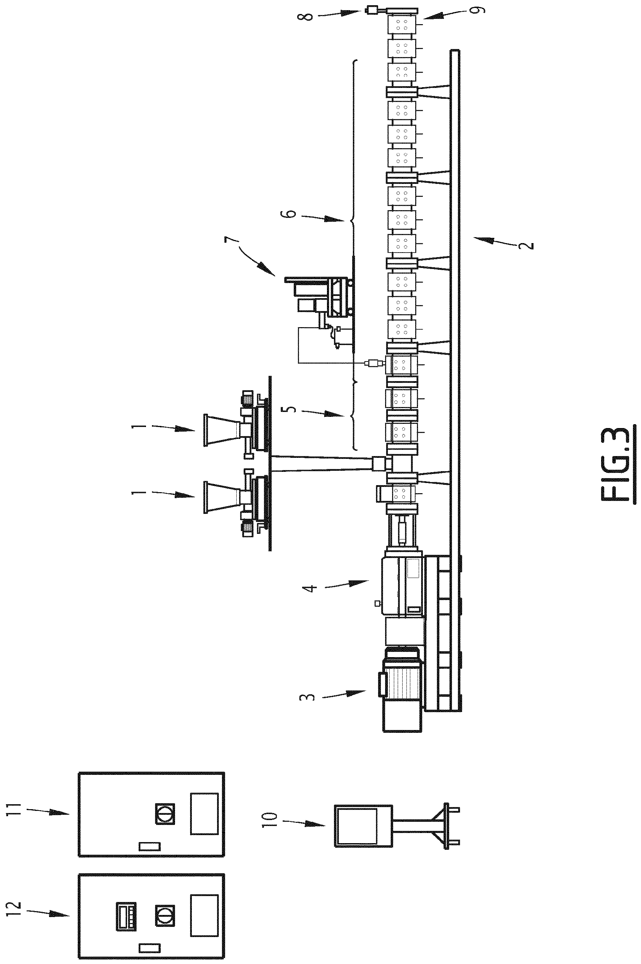

The system of Figure 3 is configured for the depolymerization of a mixture of various polymers and/or various grades of polymers.
Said mixtures are typically solid polymer wastes. They may be in the form of granules or powder, such as saw dust. Said mixtures can also include liquids like PMMA syrup (which is containing MMA oligomers and polymers dissolved in MMA), as illustrated in Figure 3 including a liquid injection.
Said polymer(s) mixture to be processed is introduced into one or more feeders 1. Feeders 1 may be of any known type such as screw, conveyors, vibration, belt and so on. Preferably gravimetric feeders are used.
Feeding rates for each feeder may be adjusted according to the present invention. The overall feeding rate may be up to 2000 kg/hour, with the feeding rate of each feeder typically comprised between 30 and 500 kg/hour, depending on product rate of requirement at commercial scale.
The fed mixture is then introduced into a heated extruder 2.
In one embodiment, the heated extruder is generally a heated twin screw extruder. The heating provides the heating energy that is consumed to melt and depolymerize the polymer mixture.
The heating may be applied internally (at the inside of the screws, by using heated screws, such as Auger screws heated by Joule effect) or outwardly (by heating the walls of the extruder) to heat the polymer mixture that is being processed.

The heating temperature may be generally higher than 400°C, typically more than 450°C, particularly comprised between 400 and 500°C. Typically, the heating temperature is adjusted at the maximal capacity of the heating device.

Typically, the heated extruder may be insulated around the outward face of its walls to avoid heat transfers and energy losses.

Suitable extruders include single screw, twin screw, multiaxial, co-rotating, counter rotation, conical type and so on. Preferably twin screw with co-rotation and intermeshing type are used from view point of high free volume in the extruder and self cleaning. Typically, extruder for pilot line TEX44 (having a 47mm screw diameter) is used and for commercial line TEX90 (having a 96.5mm screw diameter) is used, but it depends on the requested feeding rate.

TEX extruders are marketed by Japan Steel Works, and JSW can be provide bigger extruders larger than TEX90.

In a known manner, the extruder 2 is actioned by a motor 3 and gear box 4. As used herein, the gear box refers to a unit configured to

- reduce the screw speed;
- increase the torque at the screw; and/or
- divide the shaft from one shaft at motor to two shafts for the screws.

The motor 3 provides the energy required for rotating the twin screws and represents in turn the mechanical energy that is consumed by the polymer mixture.

The screw rotation speed may be typically comprised between 250 and 1000 rpm.-It can generally be adjusted depending on the size of extruder. In case of larger extruder e.g. TEX90, the screw speed range may be typically comprised between 250 and 500 rpm.

As the polymer mixtures moves along the heated extruder 2, the polymer mixture first melts in a melting zone 5 and then depolymerizes in a depolymerization zone 6.

The length of the twin screw extruder 2 is adjusted so that the polymer is fully molten and depolymerized at the exit of the extruder. If necessary, an extruder of higher length can be considered when required (e.g.) if not fully depolymerized product is collected at the exit of the extruder. Generally, when not fully polymerized product is collected, the processing temperature and hence the heating energy can be increased, or the feed rate can be decreased to achieve proper depolymerization.

At the exit of the extruder, the depolymerized product is collected in a collecting tank (not represented in Figure 3). Said collecting tank may collect solid residues exiting from the extruder and may typically comprise a condenser to collect the vaporized products such as gaseous monomers.

Typically, the system may also comprise a vacuum port such as a vent to remove air from the system, to ensure a low oxygen partial pressure in the system as it is a pyrolysis process. Figure 3 represents such vent located upstream of the feeding zone although the vent can also be located downstream of the feeding zone and/or both.

In a known manner, the system may also include a recycling loop to recycle polymers that have not been depolymerized. Typically, these are generally molten polymers or partially depolymerized PMMA and/or a PMMA syrup (MMA oligomers or PMMA dissolved in MMA) and may be fed back to the extruder before the depolymerization 6, such as at the end of the melting zone 5, through the liquid feeding apparatus 7. Typically, the liquid injection 7 is located after the formation of a molten PMMA plug.

The system may comprise various sensors for providing data on the working conditions, such as one or more temperature sensors, pressure sensors, etc..

As apparent from Figure 3, the system may include:

- One or more pressure sensors 8 measuring the polymer pressure and located at the end of the screw(s),
- one or more temperature sensors measuring the temperature of the extruder, located at each of the heating barrels. All parameters (such as Screw speed, Feed rate, Barrel temperature, Energy consumption, Polymer Pressure and so on) are indicated on the operation panel 10.

A control cabinet 11 may be included to control the heating energy and the feed rate measured.

Additionally, the motor parameters (Screw speed, motor power measured may be controlled in a motor control cabinet.

Data regarding the consumed heating energy may be calculated from the cumulated instant electricity consumption, which is divided by the time increment, or which can be calculated based on linearized cumulated electricity consumption over a certain period (for example 25, 100 or 200 data) versus time, in order to obtain an energy consumption as kWh/h, which can be converted to MJ/hr, but also once divided by the feeding rate converted to MJ/kg feed, or once corrected for the yield of the depolymerization converted to MJ/kg of crude MMA produced.

Alternatively, the heating energy [kwh] is calculated by using the heater output [%], and recorded by operation panel or/and laptop PC, according to the following relation:

$$\text{The heater energy [kwh]} = \text{Rated power [kW]} \times \text{heater output [\%]}$$

Data regarding the consumed mechanical energy may be calculated from the cumulated instant electricity consumption, which is divided by the time increment, or which can be calculated based on linearized cumulated electricity consumption over a certain period (for example 25, 100 or 200 data) versus time, in order to obtain an energy consumption as kWh/h, which can be converted to MJ/hr, but also once divided by the feeding rate converted to MJ/kg feed, or once corrected for the yield of the depolymerization converted to MJ/kg of crude MMA produced.

Alternatively, the mechanical energy [kwh] is calculated by using the motor output [%], and recorded by operation panel or/and laptop PC, according to the following relation:

$$\text{The mechanical energy [kwh]} = \text{Maximum Motor power [kW]} \times \text{motor output [\%]}$$

The sensors are connected with a control unit that is in turn connected with one or more of the feeders, the heating device and the motor. According to an embodiment, the control loop adjusts the feeding rate of the feeders and/or the screw rotation speed depending on the variation of the consumed mechanical energy that is measured. The system can also increase the operation temperature, but this usually takes more time and is not responding as fast as the mechanical energy.

The system may also generally comprise a plurality of connection ports, as required, including an inlet connection port for connecting the feeder(s) to an inlet of the extruder, and an outlet connection port to connect the outlet of the extruder to a collecting tank.

Additionally, control loops can be established to adjust one or more parameters to optimize the depolymerization process, in particular to adjust the feed rate.

Three illustrative control loops are described below in a non limitative fashion:

Illustrative control loop 1 is illustrated in Figures 6 and 7:

First, a maximum screw rotation speed at different feeding rates is established based on the construction design. A first function is then established as Max Screw Rotation as a function of the feeding rate (in kg/h). For the pilot plant (illustrated in Figure 6) the maximum screw rotation speed was 1000 rpm by construction; for a larger scale, of course higher feed rates would be used.

Then, a second set of data (the maximum heating energy versus temperature) is introduced manually into the system, based on experimental values for the designed construction.

The maximum mechanical energy and the maximum feeding rates which are defined by the construction design are also introduced as a third set of data.

According to control loop 1 (Figure 7), the pressure on the last barrel/connecting plate can be measured (optional, in dotted line). If the pressure is above a maximum pre-set value, it means that polymer is reaching the pressure sensor 8 and the feeding rate has to be decreased. If the pressure is below the limit then the next value analyzed is the heating energy. If the Energy consumed by the heaters is above a certain limit (at operating temperature), and if the screw speed is above the limit, the screw rotation is decreased and the feeding rate is decreased. Then the control loop is reinitiated. If the screw speed was not above the limit, the screw speed is increased and the feeding rate is increased before reinitiating the control loop. When the heating energy consumed is not above the maximum heating energy, then the feeding rate is increased.

The controls on the unit try to keep the temperature at the pre-set values, and will increase the heating energy supply, while the increase feeding rate will increase the mechanical energy demand.

The data input in the control loop are data averaged on several measurements. Because of the continuous evolution of the unit, the averaged data are mobile averages. For example for the pressure measurement, it is the average of past measurements as:

$$P = \frac{1}{N} * \sum_{i=1}^{i=N} Pi$$

For the screw rotation speed (RPM= Rotations per minute), similarly:

$$RMP = \frac{1}{N} * \sum_{i=1}^{i=N} RPMi$$

For the feed rate:

$$F = \frac{1}{N} * \sum_{i=1}^{i=N} Fi$$

For the Energy consumption, the value is calculated as the slope of the cumulated Energy consumption on heaters (or motor) versus time. The cumulated energy consumed is then approximated to a linear function a*t+b, where "a" is the slope of the function (in kWh/h). The linearization can be made preferably on more than 25, 100 or 200 datasets with one acquisition every second or more. The energy consumption per hour, can also be divided by the feeding rate to obtain the Gross and Net Specific energies (in kWh/kg or in MJ/kg).

An alternative control loop 2 is depicted in Figures 8A and 8B:
In this control loop, the pressure sensor is not needed. A maximum screw rotation speed is preset as in the first control loop.

The Total Energy consumed (Mechanical+Heating energy) is the control factor (Figure 8B). If the Total Energy is above a maximum, then the next control is if it is a given percentage above the maximum (for example 1.05*Emax). In such a case both feed rate and screw speed are increased and the loop is reinitiated. If it is between Emax and 1.05*Emax, then the barrel temperature is decreased, and the loop is reinitiated.

If the total energy consumed is below the maximum, then the next control is to check if it is below a given percentage of the maximum, for example 0.95*Emax. In such a case, it means that not enough energy is consumed to fully depolymerize the polymer, and the feeding rate is decreased together with the screw rotation speed. When the energy consumed is between 0.95 Emax and Emax, the barrel temperature is increased, and the loop is reinitiated.

A further alternative control loop 3 is represented in Figure 9:
In this control loop, the pressure sensor is needed. A maximum screw rotation speed is preset as in the first control loop. When the polymer come to the end of extruder, the polymer relative pressure is indicated over '0' bar. It means the polymer is not completely depolymerized in the extruder.

The depolymerization rate is also assessed by the polymer pressure sensor which is installed at end of extruder. When the polymer pressure increases over the preset value (e.g. '0' bar, but the value can be put arbitrarily), the feed rate is decreased automatically, and the screw speed will be adjusted with the feed rate. The barrel set temperature will be also increased.

Typically, when operating the system, step changes can be limited to 1 % each time, and to 30 °C/h for the temperature, 50rpm in 1 minute, and 30 kg/h in 5 minutes.

Examples

A pilot plant was set up, including

- an extruder consisting of motor 3, gear box 4, barrel and screw etc are installed on one bed;
- twin screws with co-rotation and intermeshing type;
- a heater providing the heating energy is installed around the barrels, and thermocouples for temperature control are also installed in the barrels;.
- The twin screws are installed in the barrels, and the length of screws and number of barrels can be changed freely for the depolymerization performance;
- The barrels and screws are arranged by segmented method, in order to easily exchange the parts;

The material to depolymerize is fed into the twin screw extruder at the hopper barrel by gravimetric screw feeders (measuring a weight loss). The material is melted in the melting zone mostly by shear stress from screw, then melted material is moved to the depolymerization section. The liquid MMA syrup (PMMA and MMA oligomers dissolved in MMA) can also be fed into the extruder at melting zone by liquid injection pump.

The depolymerization section uses special barrel heater which are available for maximum temperature of up to 550°C.

At the depolymerization section, the polymer is depolymerized by heater energy assisted by the shear stress from screw (mechanical energy), and transformed into the MMA monomer gas which is moved to downstream equipment.

A solid separator is included at the end of the extruder, to trap any solid that would be generated by the depolymerization, such as dust and non depolymerized PMMA. A condenser condenses the gaseous MMA produced by the depolymerization, and a pump continuously withdraws crude MMA from the condenser. The unit is also equipped with several safety devices such as pressure relief valves, pressure and temperature sensors, and the like.

PMMA mixtures in the form of crushed particles were fed. Two to four independent feeders were loaded with cast, Injection and/or extrusion PMMA.

The following standard operating conditions were set up:

Flow rate of the feeder: Total constant feed rate of 70 kg/h in standard conditions

Rotation speed of the screws: 800 rotations per minute, in standard conditions

Temperature of the screw extruder: 470 °C, in standard conditions. However, the first and last barrels are operated at lower temperature. The first barrels correspond to the melting zone and the last barrel is before a connecting plate connected to the solid separator. For example, the temperature profile from first to last barrel can be 14, 21, 250, 326, 427, 469, 470, 470, 470, 470, 470, 470, 470, 470, 470, 435, 405, and 271 °C for the last flange, in a case with an extrusion material and 70 kg/h.

All the PMMA cast, extrusion and injection samples were previously crushed to a size of about 5 to 10 mm. PVC contamination in cast material had been removed before crushing. Polyethylene protecting film has been removed also before crushing as much as possible. In the case of mixed products some contamination with other materials is nevertheless possible but estimated to below 5 wt %, and most probably below 0.5 wt %.

Sensors measured the consumed mechanical energy and heating energy on the extruder.

The energy consumption on the heaters and on the motor are recorded on the twin screws control panel.

The energy needed for the depolymerization was calculated. This energy includes:

- Specific heat to bring PMMA from room temperature to melting point (m.Cp(PMMA).$\Delta$T)
- The heat of fusion (which is low as the product is mostly amorphous): $\Delta$Hf
- Specific heat to bring PMMA to depolymerization temperature (m.Cp(PMMA).$\Delta$T)
- The heat of depolymerization: $\Delta$Hdp
- The heat of Vaporization of MMA (T): $\Delta$Hv(T)
- Specific heat to bring MMA vapors to exit temperature (m.Cp(MMA).$\Delta$T).

The total heat is brought by the heating energy of the screw and the mechanical energy of the screw, corrected for the heat loss. The heat loss is due to the low insulation of the pilot unit, but even on a commercial unit some heat loss is unavoidable. Ideally, this should also be corrected for the energy lost in the gear box and first barrel (cooled to avoid material transfer), but those values have not been recorded during the first tests.

It was found that extruded grade requires 30% less mechanical energy than cast grade of PMMA as follows :

| Entry | Grade CAST/XT / Inj | Temperature | Feed rate | Screw speed | Motor Energy | Heater Energy | Gross Motor | Gross Heater |
|---|---|---|---|---|---|---|---|---|
| | | (°C) | (kg/h) | rpm | kWh/h | kWh/h | MJ/h | MJ/h |
| 1 | Cast | 430 | 20 | 200 | 3.97 | 13.26 | 14.29 | 47.74 |
| 2 | Cast | 430 | 50 | 800 | 15.14 | 15.19 | 54.50 | 54.68 |
| 3 | Cast | 430 | 50 | 800 | 14.38 | 15.17 | 51.77 | 54.61 |
| 4 | Cast | 450 | 50 | 250 | 9.63 | 21.81 | 34.67 | 78.52 |
| 5 | Cast | 450 | 35 | 350 | 7.82 | 17.66 | 28.15 | 63.58 |
| 6 | Cast | 450 | 50 | 500 | 12.56 | 20.00 | 45.22 | 72.00 |
| 7 | Cast | 470 | 65 | 650 | 16.26 | 25.04 | 58.54 | 90.14 |
| 8 | Cast | 470 | 30 | 800 | 10.76 | 14.00 | 38.74 | 50.40 |
| 9 | Cast | 470 | 50 | 800 | 12.99 | 19.02 | 46.76 | 68.47 |
| 10 | Cast | 470 | 50 | 800 | 15.04 | 18.63 | 54.14 | 67.07 |
| 11 | Cast | 470 | 80 | 800 | 22.06 | 25.77 | 79.42 | 92.77 |

(continued)

| Entry | Grade CAST/XT / Inj | Temperature | Feed rate | Screw speed | Motor Energy | Heater Energy | Gross Motor | Gross Heater |
|---|---|---|---|---|---|---|---|---|
| 12 | Cast | 470 | 100 | 800 | 25.65 | 30.60 | 92.34 | 110.16 |
| 13 | Cast | 470 | 100 | 800 | 26.52 | 29.64 | 95.47 | 106.70 |
| 14 | Cast | 470 | 130 | 800 | 33.01 | 34.77 | 118.84 | 125.17 |
| 15 | Cast | 480 | 80 | 800 | 20.39 | 28.01 | 73.40 | 100.84 |
| 16 | Cast | 490 | 90 | 800 | 22.71 | 30.99 | 81.76 | 111.56 |
| 17 | Inj | 470 | 50 | 544 | 7.90 | 25.03 | 28.44 | 90.11 |
| 18 | mix | 470 | 50 | 544 | 8.92 | 23.60 | 32.11 | 84.96 |
| 19 | mix | 470 | 60 | 800 | 14.24 | 23.28 | 51.26 | 83.81 |
| 20 | mix | 490 | 60 | 800 | 13.95 | 24.39 | 50.22 | 87.80 |
| 21 | mix | 490 | 60 | 800 | 14.46 | 23.45 | 52.06 | 84.42 |
| 22 | XT | 470 | 50 | 544 | 7.02 | 25.71 | 25.27 | 92.56 |
| 23 | XT | 470 | 30 | 800 | 6.77 | 15.49 | 24.37 | 55.76 |
| 24 | XT | 470 | 50 | 800 | 9.59 | 21.69 | 34.52 | 78.08 |
| 25 | XT | 470 | 60 | 800 | 11.27 | 25.45 | 40.57 | 91.62 |
| 26 | XT | 470 | 80 | 800 | 14.55 | 29.07 | 52.38 | 104.65 |
| 27 | XT | 490 | 80 | 544 | 12.44 | 32.27 | 44.78 | 116.17 |

*XT= extrusion grade*
*Inj = injection grade*

Entries 4 and 6 illustrate the impact of the change of screw rotation rate. Both experiments are made with the same Cast material at 450 °C and 50 kg/h. When the screw rotation increases from 250 to 500 rpm the mechanical energy increases and the heating energy demand decreases.

A similar observation was made between entries 7 on one side and 47 and 48 on the other side, all with cast materials and at 470 °C. Entry 7 was at 65 kg/h and entries 47 and 48 were at 70 kg/h, but the screw rotation was increases from 650 to 800 rpm. Here again the mechanical energy increased and the heating energy demand is reduced.

Several other examples illustrate the invention:

Entry 16 and entry 34 illustrate the impact of increased heating energy at 90 kg/h and 800 rpm, when increasing the temperature from 470 to 490 °C. Then, the mechanical energy did not change much while the heating energy increased.

Similarly, entry 15 and entry 11, entry 33 show an increase of temperature from 470 to 480 °C, at 80 kg/h and 800 rpm on a cast material. Again, the heating energy increased while the mechanical energy slightly decreased. Entries 11 and 15 were made with exactly the same cast material.

Entries 35 to 41 illustrate what happens when the composition is changed from pure cast material to mixed streams containing cast and extrusion materials, with growing content of extrusion material up to pure extrusion material. In those experiments the feeding rate was kept constant at 70 kg/h and the screw rotation remained at 800 rpm. When the extrusion material content increases, the mechanical energy decreases, while the heating energy demand slightly increases.

To illustrate an effect of the invention, when switching from pure extrusion material (entry 26, 80 kg/h, 800 rpm, 470 °C) to pure cast material and constant heating energy, one would increase the feeding rate to 100 kg/h (entries 12,13, 31) which are at the same temperature and screw rotation speeds.

Again, from an injection material operated at 60 kg/h (entry 29, 470 °C, 800 rpm), one can switch to a cast material at the same temperature and screw rotation speed at 90 kg/h (entry 32) or at least 80 kg/h (entry 11). This represents an increase in productivity of 33 to 50 %.

| Entry | Grade | Temperature | Feed rate | Screw speed | Motor Energy | Heater Energy | Motor | Heater |
|---|---|---|---|---|---|---|---|---|
| | | (°C) | (kg/h) | rpm | kWh/h | kWh/h | MJ/h | MJ/h |
| 28 | Injection | 470 | 50 | 650 | 8 | 24.1 | 28.80 | 86.76 |
| 29 | Injection | 470 | 60 | 800 | 9.6 | 26.5 | 34.56 | 95.40 |
| 30 | Injection | 470 | 70 | 800 | 11.1 | 29.9 | 39.96 | 107.6 4 |
| | | | | | | | | |
| 31 | Cast | 470 | 100 | 800 | 25.1 | 29.6 | 90.36 | 106.5 6 |
| 32 | Cast | 470 | 90 | 800 | 22.9 | 26.6 | 82.44 | 95.76 |
| 33 | Cast | 470 | 80 | 800 | 20.9 | 24.7 | 75.24 | 88.92 |
| 34 | Cast | 470 | 90 | 800 | 22.8 | 27.4 | 82.08 | 98.64 |
| 35 | Mix (100 % Cast) | 470 | 70 | 800 | 19.6 | 24.7 | 70.56 | 88.92 |
| 36 | Mix (95 %) | 470 | 70 | 800 | 19.3 | 24.3 | 69.48 | 87.48 |
| 37 | Mix (90 %) | 470 | 70 | 800 | 18.8 | 24.2 | 67.68 | 87.12 |
| 38 | Mix (80 %) | 470 | 70 | 800 | 17.6 | 24.6 | 63.36 | 88.56 |
| 39 | Mix (70 %) | 470 | 70 | 800 | 17 | 24.3 | 61.20 | 87.48 |
| 40 | Mix (35 %) | 470 | 70 | 800 | 14.6 | 25.4 | 52.56 | 91.44 |
| 41 | Mix (0 %) | 470 | 70 | 800 | 12.6 | 25.9 | 45.36 | 93.24 |
| 42 | Extrusion | 470 | 70 | 800 | 13.1 | 27.9 | 47.16 | 100.4 4 |
| 43 | Extrusion | 470 | 70 | 800 | 12.7 | 27.4 | 45.72 | 98.64 |
| 44 | Extrusion | 470 | 70 | 800 | 12.6 | 26.9 | 45.36 | 96.84 |
| 45 | Extrusion | 470 | 70 | 800 | 12.5 | 27.2 | 45.00 | 97.92 |
| 46 | Extrusion | 470 | 70 | 800 | 12.5 | 26.9 | 45.00 | 96.84 |
| 47 | Cast | 470 | 70 | 800 | 19.3 | 23.0 | 69.48 | 82.80 |
| 48 | Cast | 470 | 70 | 800 | 18.6 | 22.8 | 66.96 | 82.08 |

The molten extruded grade has a lower viscosity than cast PMMA. The screw (Motor) provides about 30 % less energy than with Cast material.

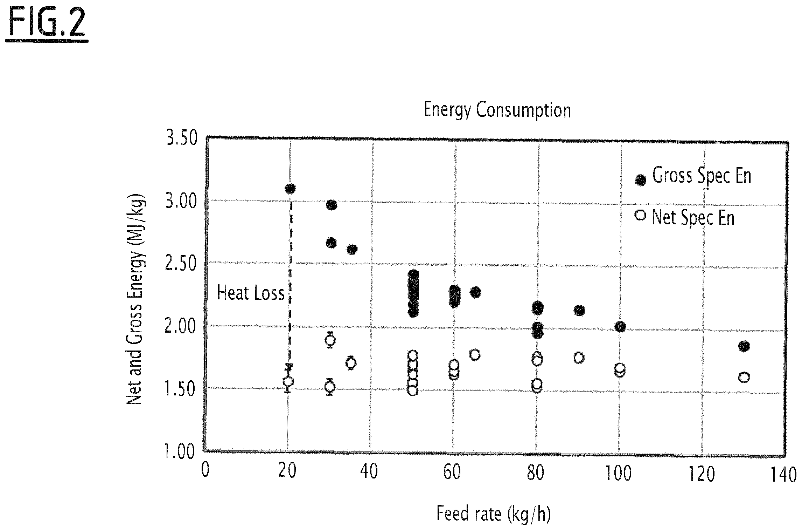

Figure 2 summarizes all the Gross Energy and Net Energy consumptions calculated in other tests. Figure 2 shows that all the treated samples are in the same range of Net Energy (1.5-1.7 MJ/kg) with multiple temperatures and feeding rates.

Data on Figure 5 show that for same heating energy, 100 kg of cast PMMA, or 70 kg of Injection PMMA (or 40 % more cast than injection PMMA); and for the same heating energy 90 kg Cast PMMA and 70 kg of extruded PMMA (or 30 % more cast than Extrusion), and a bit more than 60 kg injection PMMA can be processed..

Cast and Extruded materials do not behave the same way in the unit. The cast materials require more mechanical energy provided by the screw motor to be melted, and so have probably a higher viscosity. The heaters then provide a complement energy. The Extruded material requires less mechanical energy and so reaches more quickly the limits of what the heaters can provide. That means that the throughput with extruded material is lower than for cast material, or that we have to increase more the temperature when processing extruded materials.

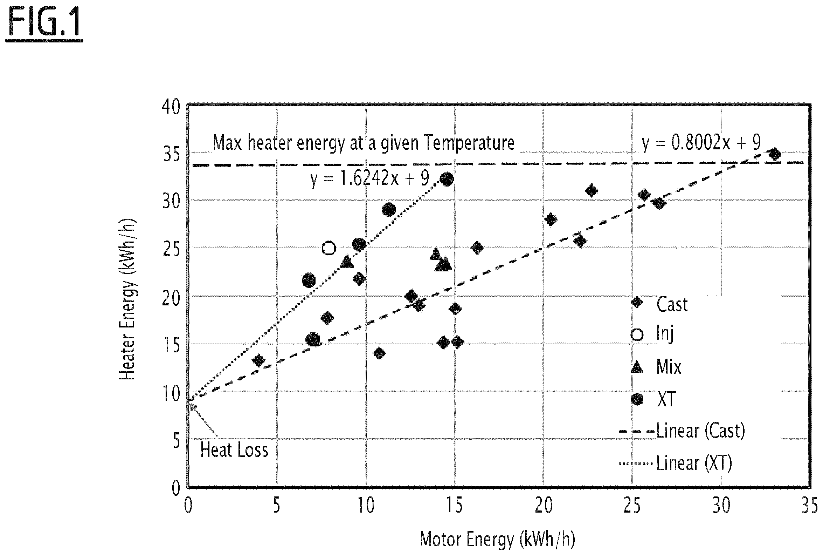

Figure 1 illustrates the Heater Energy versus the Mechanical Energy for all the tests (from 1 to 27). At higher feed rates, more energy is consumed in all cases and the products can be better differentiated. Figure 1 has been made for all tests (1 to 27), with temperature range of 430 to 490 °C, and feed rates from 20 to 130 kg/h, cast, extruded, mix of both, and injection grades.

This result of Figure 1 means that the Extruder itself can discriminate between Cast and Extruded materials.

A control loop was built to adjust several parameters of the twin screw to optimize the depolymerization parameters. Also, it was found preferable to operate the twin-screw by adjusting the screw rotation speed, which is much faster, than to change the heater temperature.

It was found favorable to operate the unit at intermediate screw rotation speed in average, and let the system adjust the screw rotation speed depending on the material processed.

It was found that the unit is limited by the heater energy and then by the total energy supplied. At a given set of conditions (temperature, screw rotation) less extruded material can be processed than cast material. As a rough estimate the limit could be 120 kg/hr with cast and 80 kg/hr with extruded material (470 °C, 800 rpm) with the current pilot. That's a 30 % reduction for extruded versus cast material, which is a significant reduction. That could be partially compensated by a smart selection of the product processed over time.

It was found that it was more appropriate to process cast material over night than during the day to get the benefit from cheaper electricity prices.

**Claims**

1. Process for depolymerizing a polymer or polymers mixture, wherein said polymer(s) may have various grades, said process comprising the steps of:

    feeding the polymer(s) to one or more processing unit(s) ;
    mechanically processing and heating the polymer(s) in the processing unit(s) configured for mechanically processing and heating said polymer(s) such as to melt and depolymerize the polymer(s);
    measuring the heating power and/or energy consumed by said one or more processing unit(s) and measuring the mechanical power and/or energy consumed by said one or more processing unit(s) ; and
    adjusting the feeding rate of the polymer(s) into the processing unit(s) depending on the measured consumed heating energy and/or the consumed mechanical energy.

2. The process according to claim 1 wherein the feeding rate is increased when the measured consumed mechanical energy increases.

3. The process according to claim 1 or 2 wherein said polymer has at least one grade requiring more mechanical energy compared with other grades of said polymer, and wherein the feeding rate is adjusted as a function of the content of the grade in the polymer or polymer mixture;

4. The process according to claim 3, wherein the feeding rate is increased when the content in said grade of said polymer in the polymer or polymers mixture increases.

5. The process according to anyone of the preceding claims wherein the polymer(s) is(are) chosen from PMMA and polystyrene.

6. The process according to claim 4 wherein the feeding rate increases when the content in cast PMMA in said PMMA or polymers mixture increases.

7. The process according to anyone of the preceding claims wherein the feeding step is carried out by a screw feeder.

8. The process according to anyone of the preceding claims wherein the processing unit(s) include a unit configured for melting the polymer(s).

9. The process according to anyone of the preceding claims wherein the processing unit(s) include a heated screw extruder configured for melting and heating the polymer(s) with heating energy and/or mechanical energy.

10. The process according to claim 9 wherein the heating of the polymers is made to a heating temperature between 400°C and 500°C.

11. A process for recycling plastics or plastics mixtures, said plastics comprising polymers, said process comprising the depolymerizing step according to anyone of claims 1 to 10.

**12.** A depolymerization system comprising

- One or more feeder(s)
- One or more processing unit(s) configured for heating and mechanically processing the polymer(s) such as to melt and depolymerize the polymer(s) ;
- a sensor for measuring the heating energy consumed by the processing unit(s) ;
- a sensor for measuring the mechanical energy consumed by the processing unit(s) ;
- a control loop configured to control the one or more feeder(s) to adjust a feeding rate as a function of the measured consumed mechanical energy and/or the measured consumed heating energy.

**13.** The depolymerization system according to claim 12 wherein the processing unit(s) include a heated screw extruder configured for melting and heating the polymer(s) with heating energy and/or mechanical energy.

**14.** The depolymerization system according to claim 12 or 13 wherein the system comprises a sensor for pressure.

**15.** The depolymerization system according to anyone of claims 12 to 14 wherein the system comprises a recycling loop to recycle polymer(s) that have not been depolymerized.

# FIG.1

EP 4 155 341 A1

# FIG.2

Energy Consumption

EP 4 155 341 A1

18

FIG.3

# FIG.4

Total Gross Energy (all data T and rpm)
T(°C) for Cast and XT

EP 4 155 341 A1

FIG.5

EP 4 155 341 A1

Max by equipment construction

FIG.6

FIG.7

A)

$E_{total} > E_{MAX}$ — NO — YES

$E_{total} < E_{MAX}*0.95$ (Step1) — NO — YES

$E_{total} > E_{MAX}*1.05$ (Step1) — NO — YES

Barrel temp. decrease

Barrel temp. increase

Feed rate increase with screw speed

Feed rate decrease with screw speed

# FIG.8 (Beginning)

B)

For example

FIG.8 (End)

**FIG.9**

Europäisches
Patentamt

European
Patent Office

Office européen
des brevets

**EUROPEAN SEARCH REPORT**

Application Number

**EP 21 30 6333**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 959 357 A (TOKUSHIGE HIROYUKI ET AL) 25 May 1976 (1976-05-25) <br> * abstract * <br> * column 2, lines 13-23 * <br> * column 2, lines 29-35 * <br> * page 2 - page 3; examples 1-6 * <br> * claims 1-4 * <br> * figure 1 * | 1-15 | INV. <br> C08J11/10 <br> B29C48/00 <br> B29B17/02 <br> C07C4/22 |
| X | Dubois Jean Luc: "Concepts of chemical recycling: part 2 Scale-up and industrial implementation", <br> , <br> 15 September 2020 (2020-09-15), pages 1-35, XP055893620, <br> DOI: 10.13140/RG.2.2.23546.52162 <br> Retrieved from the Internet: <br> URL:https://www.mmatwo.eu/wp-content/uploads/2020/10/MMAtwo-Virtual-Workshop_JL-Dubois.pdf <br> [retrieved on 2022-02-21] <br> * page 28 - page 29 * | 1-15 | |
| X <br> A | DE 31 46 194 A1 (ROEHM GMBH [DE]) 26 May 1983 (1983-05-26) <br> * page 9 - page 11 * <br> * page 11 - page 12; examples 1-3 * <br> * claims 1-11 * <br> * figure 1 * | 1,3,5, 7-11 <br> 2,4,6, 12-15 | TECHNICAL FIELDS SEARCHED (IPC) <br><br> C08J <br> C09J <br> B29C <br> B29B <br> C07C |
| A | US 4 051 212 A (GRIGAT ERNST ET AL) 27 September 1977 (1977-09-27) <br> * the whole document * | 1-15 | |

The present search report has been drawn up for all claims

| Place of search | Date of completion of the search | Examiner |
|---|---|---|
| Munich | 2 March 2022 | Schlembach, Sandra |

EPO FORM 1503 03.82 (P04C01)

## ANNEX TO THE EUROPEAN SEARCH REPORT
## ON EUROPEAN PATENT APPLICATION NO.

EP 21 30 6333

02-03-2022

| Patent document cited in search report | | Publication date | Patent family member(s) | | Publication date |
|---|---|---|---|---|---|
| US 3959357 | A | 25-05-1976 | NONE | | |
| DE 3146194 | A1 | 26-05-1983 | NONE | | |
| US 4051212 | A | 27-09-1977 | BE | 833013 A | 03-03-1976 |
| | | | DE | 2442387 A1 | 18-03-1976 |
| | | | DK | 394975 A | 05-03-1976 |
| | | | ES | 440722 A1 | 16-03-1977 |
| | | | FR | 2283766 A1 | 02-04-1976 |
| | | | GB | 1522992 A | 31-08-1978 |
| | | | GB | 1522993 A | 31-08-1978 |
| | | | IT | 1044454 B | 20-03-1980 |
| | | | JP | S557131 B2 | 22-02-1980 |
| | | | JP | S5152478 A | 10-05-1976 |
| | | | LU | 73301 A1 | 13-08-1976 |
| | | | NL | 7510298 A | 08-03-1976 |
| | | | US | 4051212 A | 27-09-1977 |

EPO FORM P0459